(19) Europäisches Patentamt
European Patent Office
Office européen des brevets

(11) EP 4 498 374 A1

(12) EUROPEAN PATENT APPLICATION

(43) Date of publication:
29.01.2025 Bulletin 2025/05

(51) International Patent Classification (IPC):
$G16B\ 20/00^{(2019.01)}$     $G16B\ 40/20^{(2019.01)}$

(21) Application number: 23188273.9

(22) Date of filing: 28.07.2023

(52) Cooperative Patent Classification (CPC):
G16B 20/00; G16B 40/20

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(71) Applicant: Siemens Healthcare Diagnostics Products GmbH
35041 Marburg (DE)

(72) Inventors:
• Schneider, Petra
53859 Niederkassel (DE)
• Siebert, Matthias
91080 Marloffstein (DE)

(74) Representative: Siemens Healthineers Patent Attorneys
Postfach 22 16 34
80506 München (DE)

(54) **EARLY CANCER DETECTION THROUGH AUTOMATED DIFFERENTIATION OF DNA METHYLATION PATTERNS FROM SAMPLES**

(57)    The present invention relates to a computer-implemented method for the analysis of a sample of a subject comprising the steps: obtaining DNA methylation data of the sample from a data storage; inputting the obtained DNA methylation data into a trained ensemble of predictive models- wherein the predictive models are based on a selection of groups of features among all features in the DNA methylation data, wherein the predictive models are trained based on a training cohort of cancer patients and healthy subjects with DNA methylation data and corresponding diagnostic, prognostic and/or predictive conclusions in the context of early detection of the cancer patients' cancer disease; and deriving a diagnostic, prognostic and/or predictive conclusion output with respect to the early detection of the cancer disease in the subject. Further envisaged are a corresponding data processing device and computer program.

FIG 1

**Description**

TECHNICAL FIELD

The present invention relates to a computer-implemented method for the analysis of a sample of a subject comprising the steps: obtaining DNA methylation data of the sample from a data storage; inputting the obtained DNA methylation data into a trained ensemble of predictive models - wherein the predictive models are based on a selection of groups of features among all features in the DNA methylation data, wherein the predictive models are trained based on a training cohort of cancer patients and healthy subjects with DNA methylation data and corresponding diagnostic, prognostic and/or predictive conclusions in the context of early detection of the cancer patients' cancer disease; and deriving a diagnostic, prognostic and/or predictive conclusion output with respect to the early detection of the cancer disease in the subject. Further envisaged are a corresponding data processing device and computer program.

BACKGROUND

Cancer development is in many cases connected to a widespread change of DNA methylation in a cell. DNA methylation is formed by the addition of a methyl group to the C5 position of the cytosine ring in cytosine residues within a 5'-cytosine-phosphate-guanine-3' (CpG) dinucleotide context in mammals. The majority of CpG sites is highly methylated in the genome with the exception of CpG islands which are largely unmethylated. Typically, genome-wide demethylation occurs during embryogenesis to form totipotent cells. This is followed by de novo methylation where tissue-specific genes undergo demethylation in their cell type of expression. DNA methylation is then maintained during DNA replication of somatic cells. Cancer-associated changes typically include a hypermethylation of CpG islands, often spanning gene promoters and first exons, i.e., an epigenetic chemical modification of genomic DNA.

Such epigenetic changes may occur early in tumorigenesis and are considered to be pervasive across a tumor type. Despite the complex nature of changes to the epigenetic situation many cancers exhibit a high degree of concordance across tissues, or within the tissue of origin (Hoadley et al., 2018, Cell 173 (2), 291-304 e296).

Lung and breast cancer are among the leading causes of death worldwide and there is a high clinical need for reliable (sensitive and specific) tests for early detection, since early treatment has the best chance of a cure.

Several approaches for automatic cancer detection based on methylation profiles have so far been described in the literature. For example, Liu et al., 2020, Ann Oncol., 31:745-759 describe multi-cancer detection and localization using methylation signatures in cell-free DNA. This preliminary study aimed at more than 50 cancer types using logistic regression but achieves insufficient sensitivity and specificity for most tumor types to be applied in a routine clinical setting. Stackpole et al., 2022, Nature Communications, 13, 5566 describe methylome sequencing of cell-free DNA for detecting and locating cancer. The authors could identify four types of methylation features, cancer(tissue)-specific hyper(hypo) methylation features, which are then integrated via an ensemble learning model. However, the currently available methylation profile-based cancer detection approaches are focused on suboptimal processes. In particular, they are not optimized for a specific type of cancer. Moreover, the described approaches aim at solving only a specific subtask, e.g., which tissue is affected if cancer is detected.

However, in order to increase the prediction accuracy for a certain type of cancer, such as lung cancer or breast cancer, a cancer type-specific model is required. Moreover, the robustness to technical and/or biological noise and the predictive power of the so far described methods are insufficient.

There is hence a need for an improved method and system for early cancer detection, in particular for lung and breast cancer.

SUMMARY

The present invention addresses these needs and provides in a first aspect a computer-implemented method for the analysis of a sample of a subject comprising the steps: (a) obtaining DNA methylation data of the sample from a data storage; (b) inputting the obtained DNA methylation data of step (a) into a trained ensemble of predictive models wherein the predictive models are based on a selection of groups of features among all features in the DNA methylation data, wherein the predictive models are trained based on a training cohort of cancer patients and healthy subjects with DNA methylation data and corresponding diagnostic, prognostic and/or predictive conclusions in the context of early detection of the cancer patients' cancer disease; and (c) deriving a diagnostic, prognostic and/or predictive conclusion output with respect to the early detection of the cancer disease in the subject.

In a further aspect the present invention relates to a computer-implemented method for providing a trained ensemble of predictive models for the analysis of a sample of a subject comprising the steps: (a) obtaining DNA methylation data of samples from a cohort of cancer patients and healthy subjects from a data storage; (b) selecting groups of features based on all features in the DNA methylation data of the cohort of step (a); (c) training an ensemble of predictive models based on the selection of groups of features obtained in step (b) with the DNA methylation data obtained in step (a) and diagnostic, prognostic and/or predictive conclusions in the context of early detection of cancer corresponding to said DNA methylation, thereby obtaining a trained ensemble of predictive models.

In yet another aspect the present invention relates to a computer-implemented method for the analysis of a sample of a subject comprising the steps: (a) obtaining DNA methylation data from samples of a cohort of cancer patients and healthy subjects from a data storage; (b) selecting groups of features based on all features in the DNA methylation data of the cohort of step (a); (c) training an ensemble of predictive models based on the selection of groups of features obtained in step (b) with the DNA methylation data obtained in step (a) and diagnostic, prognostic and/or predictive conclusions in the context of early detection of cancer corresponding to said DNA methylation, thereby obtaining a trained ensemble of predictive models; (d) obtaining DNA methylation data from a patient's sample from a data storage; (e) inputting the obtained DNA methylation data of step (d) into the trained ensemble of predictive models of step (c); and (f) deriving a diagnostic, prognostic and/or predictive conclusion output with respect to the early detection of the cancer disease in the subject. This approach, which is based on training an ensemble of classifiers and uses only a small number of features, advantageously reduces the risk of overfitting which is typically present when models are trained using very high dimensional data. It accordingly provides an optimal trade-off between underfitting (such as with classifiers with high bias, e.g., when considering too few features) and overfitting (such as with classifiers with high variance, e.g., when considering too many features). Further, the combination of multiple (weak) learners to an ensemble allows to generate highly accurate classifiers, since variance is reduced without increasing the bias. In view of the fact that the selection of features is a nondeterministic polynomial time (NP-hard) problem, suitable and sub-optimal sets of relevant features are required, in particular in a low sample-to-dimensionality setting. The present invention, by producing accurate ensemble classifiers via combining less accurate ones, on the grounds that model variance is reduced without affecting bias, provides the necessary methodology and thereby effectively increases robustness to technical and biological noise. Moreover, the claimed approach allows for the training of cancer-specific models.

In a preferred embodiment of the present invention, the selection of groups of features among all features in the DNA methylation data is based on a predefined set of features.

In another preferred embodiment the selection of groups of features among all features in the DNA methylation data is based on maximizing the discrimination of subjects with and without cancer.

In a further preferred embodiment the output is provided in the form of a metric score within a clinical decision scale.

In another preferred embodiment the method additionally comprises a step of associating a measure of uncertainty to the metric score.

It is further preferred that the training of the ensemble of predictive models additionally includes the inputting of data of a cohort of subjects affected by cancer and healthy subjects concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests.

In a further preferred embodiment of the methods of the invention, data concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests is obtained and inputted into the trained predictive models.

In further preferred embodiments the sample of a subject is a liquid biopsy sample or a tissue sample.

In yet another group of embodiments the selection of groups of features is performed with the mRMR (minimum redundancy maximum relevance), Principal Component Analysis (PCA) or t-distributed Stochastic Neighbor Embedding (t-SNE) or Uniform Manifold Approximation and Projection (UMAP) algorithm; and/or wherein the selection of groups of features is performed according to predefined information available for the methylation status, the genomic position of the methylation site or neighbouring methylation sites, or the regional methylation status.

In yet another preferred embodiment the ensemble of predictive models is an ensemble of linear discriminant analysis (LDA) classifiers.

Further, according to another preferred embodiment, one predictive model is based on one group of features.

In yet another group of embodiments the features are based on a positional and/or regional DNA methylation status.

In a further preferred embodiment, the estimate of the ensemble of predictive models is calculated as the average of the estimates of the individual predictive models.

It is particularly preferred that said cancer is a lung cancer or a breast cancer.

It is further preferred that said diagnostic, prognostic and/or predictive conclusion output comprises a cancer diagnosis and/or a subtype classification and/or a prognostic trend assessment and/or a treatment response prediction for the subject.

In a further aspect the present invention relates to a data processing device or system for the analysis of DNA methylation data, comprising means for carrying out the method as described above.

In an additional aspect the present invention relates to a computer program comprising instructions which, when the program is executed by a computer, cause the computer to carry out the method as described above.

BRIEF DESCRIPTION OF THE DRAWINGS

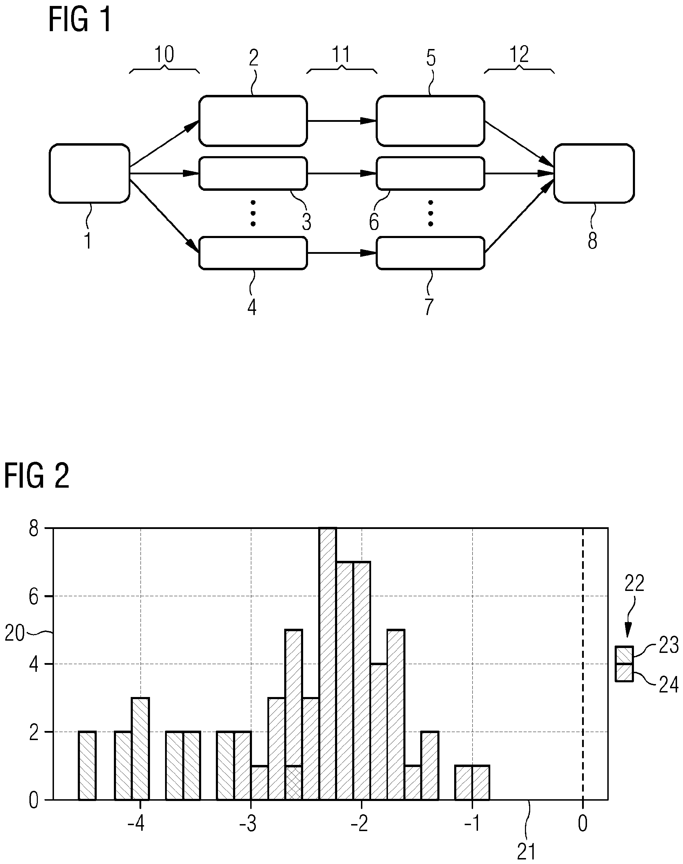

FIG. 1 shows a schematic illustration of an embodiment of the invention depicting an ensemble model for the detection of carcinogenic methylation profiles. Based on the methylation levels of selected positions in the genome (panel features (1)), a feature selection (10) is carried out. Up to N different subsets (2, 3 and 4) consisting of K methylation levels are selected (2, 3 and 4). Based on each of these reduced methylation profiles, a classifier for the detection of carcinogenic signatures in the methylation pattern is generated (classifier training (11)). The outputs of the individual classifiers (5, 6 and 7) are offset against each other to obtain the final test result (inference (12)). The final test result (prediction score (8)) is the probability of the presence of cancer in the examined patient.

FIG. 2 shows a further schematic illustration of an embodiment of the invention, namely the distribution of the variable LD1 (first linear discriminant obtained using LDA)(21) calculated from the methylation levels of five selected positions in the genome. The five markers were selected by the mRMR algorithm. The underlying methylation profiles were generated from blood samples (20) using NGS technology. The figure shows as tissue types (22) normal tissue (23) and tumor tissue (24).

DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of $\pm 20$ %, preferably $\pm 15$ %, more preferably $\pm 10$ %, and even more preferably $\pm 5$ %.

It is to be understood that the term "comprising" is not limiting. For the purposes of the present invention the term "consisting of" or "essentially consisting of" is considered to be a preferred embodiment of the term "comprising". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a computer-implemented method for the analysis of a sample of a subject comprising the steps: (a) obtaining DNA methylation data from samples of a cohort of cancer patients and healthy subjects from a data storage; (b) selecting groups of features based on all features in the DNA methylation data of the cohort of step (a); (c) training an ensemble of predictive models based on the selection of groups of features obtained in step (b) with the DNA methylation data obtained in step (a) and diagnostic, prognostic and/or predictive conclusions in the context of early detection of cancer corresponding to said DNA methylation, thereby obtaining a trained ensemble of predictive models; (d) obtaining DNA methylation data from a patient's sample from a data storage; (e) inputting the obtained DNA methylation data of step (d) into the trained ensemble of predictive models of step (c); and (f) deriving a diagnostic, prognostic and/or predictive conclusion output with respect to the early detection of the cancer disease in the subject.

The method thus starts with a step of data collection of DNA methylation data of the sample of a cohort of cancer patients and healthy subjects. The term "DNA methylation data" as used herein refers to a set of data which defines the presence or absence of a methyl group at the C5 position of the cytosine ring in the cytosine residue at a 5'-cytosine-phosphate-guanine-3' (CpG) dinucleotide site in the genomic sequence derivable from the sample of the subject. The presence of a methyl group at a CpG site in particular refers to a cytosine in a CpG dinucleotide which is methylated to form 5-methylcytosine. The absence of a methyl group at a CpG site accordingly refers to a cytosine in a CpG dinucleotide which is not methylated and thus is not present in the form of 5-methylcytosine. The presence or absence of methyl groups at CpG sites is registered with respect to genomic coordinates of said sites, wherein said coordinates are provided in the context of a suitable reference genome. The DNA methylation data is typically stored in a data storage, e.g., in a computer system, in a cloud-based server or a local server or any similar database structure which allows for localizing and retrieving data. The data may have previously been obtained experimentally, e.g., by using methylation sequencing (e.g., bisulfite sequencing or whole genome bisulfite sequencing (WGBS)), ELISA-based DNA methylation profiling, luminometric methylation assays or the use of bead arrays. Further information would be known to the skilled person or can be derived from suitable literature references such as The BLUEPRINT consortium, 2016, Nat Biotechnol., 34(7):726-37.

In specific embodiments, the DNA methylation data may alternatively be derived from a DNA methylation data database, e.g., a publicly available database or from literature repositories with database functions. Examples include The Cancer Genome Atlas (TCGA) associated databases. The database is publicly accessible over the internet, e.g., at https://www.cancer.gov/ccg/research/genome-sequencing/tcga (last visited on June 13, 2023). Further envisaged are resources as mentioned in Holm et al., 2016, Breast Cancer Res, 18(1), 27.

The data is obtained in any suitable text-based format, e.g., as CSV, BED or XML. Preferred is the BED format.

The data may be stored locally or in a cloud system or may be derived from the data storage or database on-the-fly. The step of obtaining data may, in certain embodiments, also include an update functionality which searches for changes to DNA methylation data in the database. This update may, for example, be performed within a predefined period, e.g., every week, 2, 3, 4 weeks, 2 months, 3, months, 4 months, etc.

The data may, in specific embodiments, be modified or processed, e.g., transformed into a new format, analyzed with respect to a feature of interest, or combined in any suitable way.

The DNA methylation data is obtained from at least two cohorts of persons, a cohort of cancer patients and a cohort of healthy subjects. The cohort of cancer patients may comprise patients affected by the same type of cancer, e.g., lung cancer or breast cancer. Also envisaged are cohorts of cancer patients being affected by different cancer types. A very good performance of the model can be expected if the cohort of cancer patients and healthy subjects is of similar size. Further envisaged are cohorts which alternatively or additionally comprise patients which are comparable or similar with respect to further characteristics such as distribution of age, sex, pack years (e.g., in lung cancer scenarios), demographic factors etc.

The term "cancer disease" as used herein refers to any cancer, tumor or under-controlled or uncontrolled cellular proliferation. Examples of cancer diseases or corresponding medical conditions include a stomach, colon, rectal, liver, pancreatic, lung, breast, cervix uteri, corpus uteri, ovary, prostate, testis, bladder, renal, brain/CNS, head and neck, or throat cancer, Hodgkin's disease, non-Hodgkin's lymphoma, multiple myeloma, leukemia, melanoma skin cancer, non-melanoma skin cancer, acute lymphocytic leukemia, acute myelogenous leukemia, Ewing's sarcoma, small cell lung cancer, choriocarcinoma, rhabdomyosarcoma, Wilms' tumor, neuroblastoma, hairy cell leukemia, mouth/pharynx, esophagus, larynx, kidney cancer, lymphoma, or any subtype thereof. It is preferred that the cancer disease is a lung cancer or a breast cancer. Further, the cancer disease may be a predisposition for cancer or tumor which is reflected by the presence of certain DNA methylation patterns.

In a subsequent step the DNA methylation data is analysed and groups of features based on all features in the methylation data of the cancer patient cohort and the healthy subject cohort are selected. The selection of features leads in essence to a reduction of the previous available group of all features to a reduced group of features. The term "feature" as used herein refers to the methylation level of CpG sites. Typically, the methylation levels of all measured CpG sites are included in the feature reduction. The use of feature reduction algorithms as described herein leads to a selection of CpG sites which have different levels of methylation in the cancer patient cohort and the healthy subject cohort.

In certain preferred embodiments the features are based on a positional and/or regional DNA methylation status, e.g., in the cohort of the cancer patients vs. the cohort of the healthy subjects. The term "positional DNA methylation status" as used herein refers to the binary status at a specific position in the genome, e.g., a coordinate of a genomic reference sequence. The status may accordingly be methylated or unmethylated. The term "regional DNA methylation status" as used herein refers to a summary methylation status for a larger section of a genome, e.g., 1 kb, 2 kb, 3 kb, 10 kb, 20 kb, 50 kb, 100 kb, 500 kb or more or any value in between the mentioned values. The status may be, for example, "methylated" if 50% or more of the CpG sites in the region are methylated and "unmethylated" if less than 50% of the CpG sites in the region are methylated. In a specific embodiment the summary methylation status may be an average methylation status. In further embodiments the regional DNA methylation status may be a composite methylation status, with the average methylation status as defined above as one possible characteristic. Further elements of the composite methylation status may include the methylation status of known genomic elements, e.g., CpG islands, genes, gene promoters, enhancers, transcription factor binding sites, including extensions to said sections, e.g., within 1 kb around transcription start site etc. In further specific embodiments the regional DNA methylation status may accordingly be defined with respect to known genomic elements, e.g., CpG islands, genes, gene promoters, enhancers, transcription factor binding sites, including extensions to said sections, e.g., within 1 kb around transcription start site etc. In further specific embodiments the regional DNA methylation status may correspond to the mean methylation levels of all CpG sites in a region.

The feature selection step needs to be repeated multiple times in order to generate an ensemble of classifiers using different reduced feature sets. This step may be performed with any suitable algorithm. It is preferred that the feature selection is performed with a minimum redundancy - maximum relevance (mRMR) algorithm (further details on the usage of this algorithm would be known to the skilled person and ca be derived from suitable references such as Ding and Peng, 2005, J Bioinform Comput Biol., 3(2):185-205). This algorithm is based on the task of selecting among a big number of features a small sub-group which is sufficient for achieving the maximum level of accuracy for the task, e.g., the early prediction of a cancer disease. The mRMR algorithm accordingly seeks to identify a small set of features that, together, have the maximum possible predictive power. Briefly, the algorithm is used iteratively and at each iteration the feature is selected that has maximum relevance with respect to a target variable and minimum redundancy with respect to the features that have been selected at previous iterations. According to the present invention, the target variable is the presence of a cancer disease (yes/no). At each iteration the mRMR algorithm provides a score for each feature to be evaluated, wherein the feature with the highest score is selected. The calculation of the score may, for example, be based on statistical calculations, e.g., F-statistics and correlations such as Pearson correlation. Alternatively, information theoretic measures like the mutual information can be used. It is further envisaged that in specific embodiments, in order to generate different feature sets with a repeated use of the mRMR algorithm, the features that had the highest relevance in relation to the target variable are ignored. In further embodiments, the algorithm may be operated on different bootstrap samples (i.e., samples with replacement) of the complete training data.

Accordingly, the features determined comprise the individual elements of the methylation profile, in particular the methylation stages of selected positions in the genome. The mRMR algorithm thus selects minimally correlated positions in the genome whose methylation states are maximally predictive of the existence of a tumor.

It is particularly preferred that the selection of groups of features among all features in the DNA methylation data is based on maximizing the discrimination of subjects with and without cancer.

The feature selection may, in certain embodiments of the present invention, also be performed with data transformation methods which yield dimensionality reduction. Accordingly, there is no selection of (groups of) predefined features (e.g., position X on chromosome Y is methylated or not), but new dimensions or features are defined from which a selection can be made. The selection may, for example, be limited to the selection of features corresponding to the K dimensions with the greatest variance. Examples of such data transformation methods include Principal Component Analysis (PCA), t-distributed Stochastic Neighbor Embedding (t-SNE) and Uniform Manifold Approximation and Projection (UMAP), which are described in more detail herein below.

PCA is a technique for analyzing large datasets containing a high number of dimensions/features per observation, increasing the interpretability of data while preserving the maximum amount of variation, and enabling the visualization of multidimensional data. PCA allows to reduce the dimensionality of data, which is accomplished by linearly transforming the data into a coordinate system where the main variance in the data can be described with fewer dimensions than initially. Details on the usage of this algorithm would be known to the skilled person and can be derived from suitable references such as Ma and Dai, 2011, Briefings in Bioinformatics, 12, 6, 714-722; or Huang et al., 2022, Communications Biology, 5, 719.

A further alternative algorithm is t-distributed Stochastic Neighbor Embedding (t-SNE). t-SNE is a statistical method for visualizing high-dimensional data by giving each datapoint a location in a two or three-dimensional map. It typically models each high-dimensional object by a two- or three-dimensional point such that similar objects are modeled by nearby points and dissimilar objects are modeled by distant points with high probability. t-SNE typically constructs a probability distribution over pairs of high-dimensional objects such that similar objects are assigned a higher probability while dissimilar points are assigned a lower probability. Subsequently, t-SNE defines a similar probability distribution over the points in the low-dimensional map, then minimizes the Kullback-Leibler divergence between the two distributions with respect to the locations of the points in the map. Details on the usage of this algorithm would be known to the skilled person and can be derived from suitable references such as Huang et al., 2022, Communications Biology, 5, 719.

Another suitable approach for feature selection is based on the algorithm Uniform Manifold Approximation and Projection (UMAP). UMAP is a nonlinear dimensionality reduction technique that can be used for visualization. It typically assumes that the data is uniformly distributed on a locally connected Riemannian manifold and that the Riemannian metric is locally constant or approximately locally constant. Details on the usage of this algorithm would be known to the skilled person and can be derived from suitable references such as Huang et al., 2022, Communications Biology, 5, 719.

The present invention also envisages the use of feature selection via the Boruta package, e.g., as described in Kursa and Rudnicki, 2010, J Stat Soft; 36(11), 1-13; as well as FIt-SNE, TriMap, PaCMAP, ForceAtlas2, and PHATE as described in Huang et al., 2022, Communications Biology, 5, 719.

In further embodiments the selection of groups of features among all features in the DNA methylation data is based on a predefined set of features. Such a set of predefined features may be based on independently available information on the relevance of certain features or groups of features, e.g., from previous experiments, literature sources, database entries or the like. The predefined information may include, for example, the methylation status, the genomic position of the methylation site or information on neighbouring methylation sites, or information on the regional methylation status. The predefined information may, in further embodiments, be updated or compared with updated database entries or literature sources during the performance of the method.

This additional information may be used as supplementary data for the feature selection procedure or may be compared and aligned with the result of the feature selection step in order to improve the feature selection outcome. For example, the features selected via the feature selection as described herein may be supplemented with additional features that are already known to be relevant. Additional information may further or alternatively be used for a pre-selection of all measured features, e.g., by filtering those features lying in pre-selected areas, e.g., in or around (certain) CpG islands, genes and/or other genomic elements.

In a subsequent step of the method predictive models are trained based on the selection of groups of features obtained in the previous step with the DNA methylation data and diagnostic, prognostic and/or predictive conclusions in the context of early detection of cancer corresponding to said DNA methylation. This allows to obtain a group or an ensemble of distinct trained predictive models. Accordingly, the present invention envisages the implementation of a concept of ensemble learning. Each single element of said ensemble is based on one previously selected group of features as described above.

It is particularly preferred that one predictive model is based on one group of features.

The training of the predictive models or classifiers is accordingly linked to the selected groups of features, wherein said features are associated with diagnostic, prognostic and/or predictive conclusions in the context of early detection of cancer.

The term "diagnostic, prognostic and/or predictive conclusions" as used herein refers to information on medical or therapeutic consequences of certain features of the DNA methylation data. For example, if a certain DNA methylation status which is found in the data of the cohort of cancer patients (but not in the data of the cohort of healthy patients) is connected to a cancer disease, this connection or link may be translated into a diagnostic output, e.g., a classification as carcinogenic. Corresponding information may, for example, be derived from suitable literature sources or database entries associated with the DNA methylation data. Similarly, if certain DNA methylation patterns are connected to early symptoms of a cancer disease this connection or link may be translated into a prognostic output, e.g., reflected by a classification as potentially carcinogenic or carcinogenic in an early stage or the like. Corresponding information may, for example, be derived from suitable literature sources or database entries associated with the associated DNA methylation data. Also, should certain DNA methylation patterns be connected to therapeutic suggestions or instructions in the context of a cancer disease, this connection or link may be translated into a predictive output, e.g., reflected by a corresponding classification. Corresponding information may, for example, be derived from suitable literature sources or database entries associated with the DNA methylation data.

According to the present invention the trained predictive models are classifiers based on certain groups of features previously selected by the feature selection step as described herein. A preferred classifier is based on the Linear Discriminant Analysis (LDA) procedure. LDA is a technique for dimensionality reduction problems as preprocessing step for pattern classification applications. The main purpose of the algorithm is the reduction of dimensions by removing redundant and dependent features from a higher dimensional space to a space with lower dimensions. Typically, LDA is used in the context of the present invention in classifier mode. The algorithm may accordingly be used to distinguish between two or more groups that are described by different variables. The algorithm may accordingly calculate a weighted sum of the descriptive variables used in the form:

$$\text{LD1} = a_0 + a_1 x_1 + a_2 x_2 + \ldots + a_K x_K$$

According to the invention variable $x_i$ may represent the methylation levels of selected positions in the genome, e.g., CpG sites. Parameter K may indicate the number of diagnostic markers previously selected, e.g., by the mRMR algorithm as described above, or further alternatives as mentioned herein. To determine the discriminant function LD1, two criteria are typically considered and their ratio optimized:

    1. variance of class-specific mean values (this value is maximized); and

    2. variance of data within the classes (this value is minimized)

Thereby parameters of the model are adjusted in a way that the separation of the feature groups is optimal. By identifying a suitable limit value for LD1, an automatic distinction can be made between different feature groups.

An example of the results obtained with the LDA procedure based on the methylation levels of five selected positions in the genome is provided in Fig. 2.

The present invention also envisages the use of alternative classifiers such as Support Vector Machines (SVM), e.g., as described in Shihong et al., 2003, Applied Mathematics, 18, 332-342.

In further specific embodiments, the training of the ensemble of predictive models additionally includes the inputting of data of a cohort of subjects affected by cancer concerning one or more of: age, sex, race, characteristics of cancer phenotypes, histologic characteristics, stage of development of cancer, histologic subtypes, or detectable molecular changes. Such molecular changes may include changes of a subject's or a cohort of subjects' transcriptome, metabolome, proteome, glycome or lipidome in comparison to a control or reference transcriptome, metabolome, proteome, glycome or lipidome, e.g., obtained from a healthy subject, or data on biomarkers derived from medical images, pathology images, and/or laboratory tests.

Importantly, the present invention envisages that merely data may be used which are available for the specific patient cohort analyzed. However, additional information, e.g., from literature databases, such as PubMed or the like, can be employed to select a subset of data, e.g., metabolites, that have already been associated with the cancer disease.

Also envisaged are data on the hospital where the patient and a healthy subject was examined, or the laboratory where the analysis was performed, health records including, for example, a history of earlier diseases or previous diagnoses etc. The data may be provided for the entire cohorts and introduced in the training as additional features. These data may be used as additional variables in the individual classifiers or, preferably, they are combined in a downstream classifier with the methylation-pattern-based prediction score obtained with the method of the invention.

After the training of the classifiers with selected features from DNA methylation data derived from samples of a cohort of cancer patients and healthy subjects to yield predictive models a trained ensemble of predictive models is obtained. This number of ensemble members corresponds to the number of groups of features selected in step (b) as described above.

The trained ensemble of predictive models may be used for the analysis of a sample of patient or target subject.

This analysis may, in one embodiment, be part of the above mentioned method as additional method step. Accordingly, the method envisages as step (d) obtaining DNA methylation data from a patient's sample from a data storage.

The data may, previous to the storage in a data storage, have been obtained and processed according to procedures as mentioned herein. In certain embodiments, obtaining DNA methylation data from a subject or patient may additionally include a preparation step for nucleic acids, the enrichment for genomic regions of interest, e.g., previously designated sequences, genomic regions or genes of interest etc.

According to the present invention the DNA methylation data may have been obtained from a sample derived from a subject or group of subjects. The term "sample" as used herein may accordingly have been obtained from a healthy subject or a cancer patient or a cohort of such subjects or patients as described herein above, i.e., a patient or subject whose disease status has been clarified before or independently. The sample may further have been derived from any patient or subject afflicted by a cancer disease or suspected to be afflicted by a cancer disease, or from any other subject, e.g., for control or reference purposes or from any subject independent of suspicion of being afflicted by a cancer disease, e.g., for a cancer screening. In certain embodiments, the sample is a tumor sample, i.e., the nucleic acids may be extracted from a tumor of a subject, in other embodiments the sample may be a tissue, urine, blood, semen, liquor, plasma, serum or other sample. Particularly preferred are liquid biopsy samples derived from blood/urine or other body fluids or tissue samples. Also envisaged is to make use of previously deposited samples.

DNA methylation data derived from such patient samples from a data storage are then inputted into the trained ensemble of predictive models as described above. This typically generates outputs of the individual classifiers or predictive models in the ensemble. These outputs are offset against each other in an inference step to obtain a final test result, e.g., in the form of a prediction score.

In certain preferred embodiments, in addition to the input of DNA methylation data derived from patient samples also data concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests is obtained from a patient or target subject. These data may be used as additional variables in the individual classifiers or, preferably, they are combined in a downstream classifier with the methylation-pattern-based prediction score obtained with the method of the invention.

The inference from the trained ensemble of predictive models accordingly produces an output for inputted DNA methylation data in the context of the selected and classified groups of features, allowing to derive a diagnostic, prognostic and/or predictive conclusion with respect to the early detection of the cancer disease in the target subject. In other words, the trained ensemble of predictive models produces an output which links a diagnosis, prognosis and/or therapeutic instruction to the inputted DNA methylation data. For example, the output of an individual predictive model may be the probability of the presence of a malignant tumor in the patient's body. In certain preferred embodiments, the overall inference results or the overall estimate of the ensemble of predictive models may be calculated as the average of the results of the individual predictive models.

In certain embodiments, the output may, for example, be provided as report or alert.

It is preferred that the diagnostic and/or therapeutic conclusion output comprises a disease subtype classification. For example, the trained ensemble of predictive models may provide a result, which allows to classify the cancer disease according to its subtype. Furthermore, the conclusion output may comprise a prognostic trend assessment. The output may, for example, be based on a previous diagnosis which can, according to certain embodiments, be modified by a prognostic trend definition. Likewise, a treatment response prediction may be provided as outcome. Accordingly, the target subject may receive a statement on the feasibility of certain treatment procedures.

The present invention further envisages in a preferred embodiment that the output is provided in the form of a metric score within a clinical decision scale. The term "metric score" as used herein refers to a graduation scheme which is part of the prediction performed by the trained ensemble of predictive models. This graduation scheme is provided as metric classification embedded within a clinical or therapeutic decision scale. For example, the severity of a cancer disease may be classified on a scale from 1 to 10, e.g., 1 being very mildly severe, whereas 10 is strongly severe. This diagnosis may subsequently lead to a corresponding therapeutic decision. Alternatively, the output may be a value for the likelihood of, for example, a 6-month survival. In a further example, the output may be a prediction of the time until recurrence of the cancer disease etc. Likewise, the predictive output may be provided as metric score, e.g., a certain dosage may be provided in a metric scale from 1 to 10 times a normalized dose 1, as known to the skilled person, or a radiation therapy may be suggested with a metric graduation from strength 1 to 10 based on a normalized standard strength 5 as known to the skilled person.

In a further specific step, the diagnostic, prognostic, or predictive output may additionally be finetuned or modified by associating a measure of uncertainty to the metric score as described herein. The term "measure of uncertainty" as used herein refers to a statistical factor which reflects the accuracy of the prediction of the ensemble by considering the similarity between the current DNA methylation situation of a target subject vis-à-vis the DNA methylation situation of the cohort of subjects yielding the training data set. The statistical factor may, for example, be derived from a measurement of the similarity based on the selected features and via the similarity the DNA methylation levels of a target subject with the DNA methylation levels of the training cohort.

In a further embodiment the statistical factor may be derived from the measurement of patient similarity, e.g., with respect to age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests etc. A measurement of similarity may, for example, be performed with a taxicab or Manhattan geometry, wherein the distance between two points is measured as sum of absolute differences of Cartesian coordinates. For example, in case a target subject's DNA methylation data yields a DNA methylation pattern, which is reflected by DNA methylation data derivable from subjects of the training cohort, more preferably by data of subjects which have been diagnosed with cancer, the likelihood of the target subject having cancer is high. In consequence the uncertainty would be low.

In a further aspect the present invention relates to a computer-implemented method for the analysis of a sample of a subject comprising the steps: (a) obtaining DNA methylation data of the sample from a data storage; (b) inputting the obtained DNA methylation data of step (a) into a trained ensemble of predictive models wherein the predictive models are based on a selection of groups of features among all features in the DNA methylation data, wherein the predictive models are trained based on a training cohort of cancer patients and healthy subjects with DNA methylation data and corresponding diagnostic, prognostic and/or predictive conclusions in the context of early detection of the cancer patients' cancer disease; and (c) deriving a diagnostic, prognostic and/or predictive conclusion output with respect to the early detection of the cancer disease in the subject.

Step (a) essentially corresponds to the step of obtaining DNA methylation data of the sample of a target subject or patient from a data storage as defined herein above. Further the trained ensemble of predictive models is an ensemble of predictive models as described herein above in detail. Accordingly, a trained ensemble of predictive models as described herein is used for the inputting step of this method. Similarly, the step (c) of the method essentially corresponds to the inference step as described in detail herein.

In another aspect the present invention relates to a computer-implemented method for providing a trained ensemble of predictive models for the analysis of a sample comprising the steps: (a) obtaining DNA methylation data of samples from a cohort of cancer patients and healthy subjects from a data storage; (b) selecting groups of features based on all features in the DNA methylation data of the cohort of step (a); (c) training an ensemble of predictive models based on the selection of groups of features obtained in step (b) with the DNA methylation data obtained in step (a) and diagnostic, prognostic and/or predictive conclusions in the context of early detection of cancer corresponding to said DNA methylation, thereby obtaining a trained ensemble of predictive models.

Steps (a) and (b) essentially correspond to steps (a) and (b) of the computer-implemented method for the analysis of a sample of a subject as described herein. Further, step (c) of this method essentially corresponds to step (c) of the computer-implemented method for the analysis of a sample of a subject as described herein. The method accordingly provides a trained ensemble of predictive models for the analysis of a sample. The present invention also envisages this trained ensemble of predictive models as hardware- and/or software implementation.

In a further aspect the present invention relates to a computer-implemented method for the analysis of DNA methylation data. The method may be implemented on any suitable storage or computer platform, e.g., be cloud-based, internet-based, intra-net based or present on local computer or cell phones etc.

In another aspect the present invention relates to a data processing device comprising means for carrying out the computer-implemented method as defined above. The device comprises means for carrying out any one or more steps of the computer-implemented method of the present invention as mentioned herein above. Accordingly, any of the computer-implemented methods described herein may be totally or partially performed with a computer system including one or more processor(s), which can be configured to perform the steps. Accordingly, some of the present embodiments are directed to computer systems configured to perform the steps of any of the computer-implemented methods described herein, potentially with different components performing respective steps or a respective group of steps. Corresponding steps of methods may further be performed at the same time or in a different order. Additionally, portions of these steps may be used with portions of other steps from other methods. Also, all or portions of a step may be optional. Additionally, any of the steps of any of the methods can be performed with models, circuits, or other means for performing these steps.

Also envisaged is a computer program comprising instructions which, when the program is executed by a computer, cause the computer to carry out any of the computer-implemented methods of the invention as defined herein or any one or more computerizable steps of the methods of the present invention as mentioned herein.

Also envisaged is the provision of a computer-readable storage medium comprising a computer program product as defined above. The computer-readable storage medium may be connected to a server element, or be present in a cloud structure, or be connected via internet or intranet to one or more database structures, or client databases etc.

Any of the software components or computer programs or functions described herein may be implemented as software code to be executed by a processor using any suitable computer language such as, for example, Java, Python, JavaScript, VB.Net, C++, C#, C, Swift, Rust, Objective-C, Ruby, PHP, or Perl using, for example, conventional or object-oriented techniques. The software code may be stored as a series of instructions or commands on a computer readable medium for storage and/or transmission, suitable media include random access memory (R_AM), a read only memory (ROM), a magnetic medium such as a hard-drive, or an optical medium such as a compact disk (CD) or DVD (digital versatile disk), flash memory, and the like. The computer readable medium may be any combination of such storage or transmission devices. Such programs may also be encoded and transmitted using carrier signals adapted for transmission via wired, optical, and/or wireless networks conforming to a variety of protocols, including the Internet. As such, a computer readable medium according to the present invention may be created using a data signal encoded with such programs. Computer readable media encoded with the program code may be packaged with a compatible device or provided separately from other devices (e.g., via internet download). Any such computer readable medium may reside on or within a single computer program product (e.g., a hard drive, a CD, or an entire computer system), and may be present on or within different computer program products within a system or network. A computer system may include a monitor, printer, or other suitable display for providing any of the results mentioned herein to a user.

## Claims

1. A computer-implemented method for the analysis of a sample of a subject comprising the steps:

   (a) obtaining DNA methylation data of the sample from a data storage;
   (b) inputting the obtained DNA methylation data of step (a) into a trained ensemble of predictive models

       - wherein the predictive models are based on a selection of groups of features among all features in the DNA methylation data,
- wherein the predictive models are trained based on a training cohort of cancer patients and healthy subjects with DNA methylation data and corresponding diagnostic, prognostic and/or predictive conclusions in the context of early detection of the cancer patients' cancer disease; and

(c) deriving a diagnostic, prognostic and/or predictive conclusion output with respect to the early detection of the cancer disease in the subject.

2. A computer-implemented method for providing a trained ensemble of predictive models for the analysis of a sample of a subject comprising the steps:

(a) obtaining DNA methylation data of samples from a cohort of cancer patients and healthy subjects from a data storage;
(b) selecting groups of features based on all features in the DNA methylation data of the cohort of step (a);
(c) training an ensemble of predictive models based on the selection of groups of features obtained in step (b) with the DNA methylation data obtained in step (a) and diagnostic, prognostic and/or predictive conclusions in the context of early detection of cancer corresponding to said DNA methylation, thereby obtaining a trained ensemble of predictive models.

3. A computer-implemented method for the analysis of a sample of a subject comprising the steps:

(a) obtaining DNA methylation data from samples of a cohort of cancer patients and healthy subjects from a data storage;
(b) selecting groups of features based on all features in the DNA methylation data of the cohort of step (a);
(c) training an ensemble of predictive models based on the selection of groups of features obtained in step (b) with the DNA methylation data obtained in step (a) and diagnostic, prognostic and/or predictive conclusions in the context of early detection of cancer corresponding to said DNA methylation, thereby obtaining a trained ensemble of predictive models;
(d) obtaining DNA methylation data from a patient's sample from a data storage;
(e) inputting the obtained DNA methylation data of step (d) into the trained ensemble of predictive models of step (c); and
(f) deriving a diagnostic, prognostic and/or predictive conclusion output with respect to the early detection of the cancer disease in the subject.

4. The computer-implemented method of any one of claims 1 to 3, wherein the selection of groups of features among all features in the DNA methylation data is based on a predefined set of features.

5. The computer-implemented method of any one of claims 1 to 3, wherein the selection of groups of features among all features in the DNA methylation data is based on maximizing the discrimination of subjects with and without cancer.

6. The computer-implemented method of any one of claims 1, 3, 4 or 5, wherein the output is provided in the form of a metric score within a clinical decision scale.

7. The computer-implemented method of claim 6, wherein the method additionally comprises a step of associating a measure of uncertainty to the metric score.

8. The computer-implemented method of any one of claims 2 to 7, wherein the training of the ensemble of predictive models additionally includes the inputting of data of a cohort of subjects affected by cancer and healthy subjects concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests.

9. The computer-implemented method of claim 1 or 4 to 7, wherein data concerning one or more of: age, sex, race, characteristics of cancer phenotypes, histologic characteristics, stage of development of cancer, histologic subtypes, detectable molecular changes, preferably on the level of the transcriptome, metabolome, proteome, glycome or lipidome, biomarkers derived from medical images, pathology images, and/or laboratory tests is obtained and inputted into the trained predictive models.

10. The computer-implemented method of any one of claims 1 to 9, wherein the sample of a subject is a liquid biopsy sample or a tissue sample.

11. The computer-implemented method of any one of claims 1 to 10, wherein the selection of groups of features is performed with the mRMR (minimum redundancy maximum relevance), Principal Component Analysis (PCA) or t-distributed Stochastic Neighbor Embedding (t-SNE) or Uniform Manifold Approximation and Projection (UMAP) algorithm; and/or wherein the selection of groups of features is performed according to predefined information available for the methylation status, the genomic position of the methylation site or neighbouring methylation sites, or the regional methylation status.

12. The computer-implemented method of any one of claims 1 to 11, wherein the ensemble of predictive models is an ensemble of linear discriminant analysis (LDA) classifiers.

13. The computer-implemented method of any one of claims 1 to 12, wherein one predictive model is based on one group of features.

14. The computer-implemented method of any one of claims 1 to 13, wherein the features are based on a positional and/or regional DNA methylation status.

15. The computer-implemented method of any one of claims 1 to 14, wherein the estimate of the ensemble of predictive models is calculated as the average of the estimates of the individual predictive models.

16. The computer-implemented method of any one of claims 1 to 15, wherein said cancer is a lung cancer or a breast cancer.

17. The computer-implemented method of any one of claims 1 to 16, wherein said diagnostic, prognostic and/or predictive conclusion output comprises a cancer diagnosis and/or a subtype classification and/or a prognostic trend assessment and/or a treatment response prediction for the subject.

18. A data processing device or system for the analysis of DNA methylation data, comprising means for carrying out the method of any one of claims 1 to 17.

19. A computer program comprising instructions which, when the program is executed by a computer, cause the computer to carry out the method of any one of claims 1 to 17.

## FIG 1

## FIG 2

Europäisches
Patentamt

European
Patent Office

Office européen
des brevets

# EUROPEAN SEARCH REPORT

Application Number

**EP 23 18 8273**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MENG YAJIE ET AL: "HFS-SLPEE: A Novel Hierarchical Feature Selection and Second Learning Probability Error Ensemble Model for Precision Cancer Diagnosis", FRONTIERS IN CELL AND DEVELOPMENTAL BIOLOGY, vol. 9, 30 June 2021 (2021-06-30), XP055977686, DOI: 10.3389/fcell.2021.696359 Retrieved from the Internet: URL:https://www.frontiersin.org/articles/10.3389/fcell.2021.696359/full> * the whole document * | 1-19 | INV. G16B20/00 G16B40/20 |
| X | CN 116 356 021 A (ZHONGSHAN HOSPITAL FUDAN UNIV ET AL.) 30 June 2023 (2023-06-30) * abstract * | 1-19 | |
| X | STACKPOLE MARY L. ET AL: "Cost-effective methylome sequencing of cell-free DNA for accurately detecting and locating cancer", NATURE COMMUNICATIONS, vol. 13, no. 1, 1 January 2022 (2022-01-01), pages 1-12, XP093097014, DOI: 10.1038/s41467-022-32995-6 Retrieved from the Internet: URL:https://www.nature.com/articles/s41467-022-32995-6> * the whole document * | 1-19 | TECHNICAL FIELDS SEARCHED (IPC) G16B |

The present search report has been drawn up for all claims

| Place of search | Date of completion of the search | Examiner |
|---|---|---|
| Munich | 10 January 2024 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

2

## ANNEX TO THE EUROPEAN SEARCH REPORT
## ON EUROPEAN PATENT APPLICATION NO.

EP 23 18 8273

10-01-2024

| Patent document cited in search report | Publication date | Patent family member(s) | Publication date |
|---|---|---|---|
| CN 116356021 A | 30-06-2023 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HOADLEY et al.** *Cell*, 2018, vol. 173 (2), 291-304, e296
- **LIU et al.** *Ann Oncol.*, 2020, vol. 31, 745-759
- **STACKPOLE et al.** *Nature Communications*, 2022, vol. 13, 5566
- *Nat Biotechnol.*, 2016, vol. 34 (7), 726-37
- **HOLM et al.** *Breast Cancer Res*, 2016, vol. 18 (1), 27
- **DING** ; **PENG**. *J Bioinform Comput Biol.*, 2005, vol. 3 (2), 185-205
- **MA** ; **DAI**. *Briefings in Bioinformatics*, 2011, vol. 12 (6), 714-722
- **HUANG et al.** *Communications Biology*, 2022, vol. 5, 719
- **KURSA** ; **RUDNICKI**. *J Stat Soft*, 2010, vol. 36 (11), 1-13
- **SHIHONG et al.** *Applied Mathematics*, 2003, vol. 18, 332-342